# FASCICULE DE BREVET EUROPEEN

(11) **EP 1 044 652 B1**
(45) Date de publication et mention de la délivrance du brevet: **14.06.2006**
(21) Numéro de dépôt: 00401016.1
(22) Date de dépôt: 12.04.2000
(51) Int. Cl.: A61B 10/00, C12M 1/30

(54) **Flacon de préparation d'une suspension cytologique**
Fläschchen zur Herstellung einer zytologischen Suspension
Vial for preparing a cytological suspension

(30) Priorité: 14.04.1999 FR 9904664
(43) Date de publication de la demande: 18.10.2000
(73) Titulaire: Peltier, Eric, 92140 Clamart (FR)
(72) Inventeur: Peltier, Eric, 92140 Clamart (FR)
(74) Mandataire: Habasque, Etienne J. Jean-François

(56) Documents cités:
- EP-A- 0 175 326
- US-A- 3 815 580
- US-A- 3 828 765
- US-A- 4 439 319
- US-A- 4 633 886
- US-A- 5 422 273
- US-A- 5 833 928

## Description

La présente invention concerne un flacon de préparation d'une suspension cytologique à base de fixateur.

De tels flacons sont utilisés dans l'état de la technique, pour préparer des suspensions cytologiques cervicales ou vaginales à analyser.

Les prélèvements cervicaux ou vaginaux sont effectués par des praticiens à l'aide de brosses spécifiques fixées de façon détachable sur des manches de manipulation.

Une fois le prélèvement effectué, le praticien plonge la brosse dans le flacon et détache celle-ci du manche, de manière à permettre aux cellules prélevées de se déposer dans le fixateur.

Cependant, des composants indésirables peuvent également se déposer dans le fixateur, comme par exemple des débris récupérés par la brosse lors du prélèvement (mucus, agrégations, etc..), des squames issus du praticien et qui se déposent dans le flacon notamment lors de la manipulation de la brosse pour la détacher du manche, etc..

Or, ces composants peuvent être très gênants lors de l'analyse ultérieure de la suspension.

le doument US-A-3 828 765 décrit un flacon de préparation d'une suspension cytologique correspondant au préambule de la revendication 1.

Le but de l'invention est donc de résoudre ces problèmes.

A cet effet, l'invention a pour objet un flacon de préparation d'une suspension cytologique à base de fixateur, ce flacon étant muni d'une ouverture destinée à recevoir une brosse de prélèvement cytologique fixée de façon détachable sur un manche de manipulation, l'ouverture du flacon comporte des moyens de butée pour la brosse, permettant de bloquer celle-ci dans le flacon et de la détacher du manche et d'un voile (6) obturant l'ouverture (5) du flacon, ledit voile étant muni d'un trou (7) de passage de la brosse de prélèvement (3), les bords qui délimitent ce trou formant lesdits moyens de butée, caractérisé en ce qu'au moins une portion du voile (6) est ajourée (8) pour filtrer la suspension lors du versement.

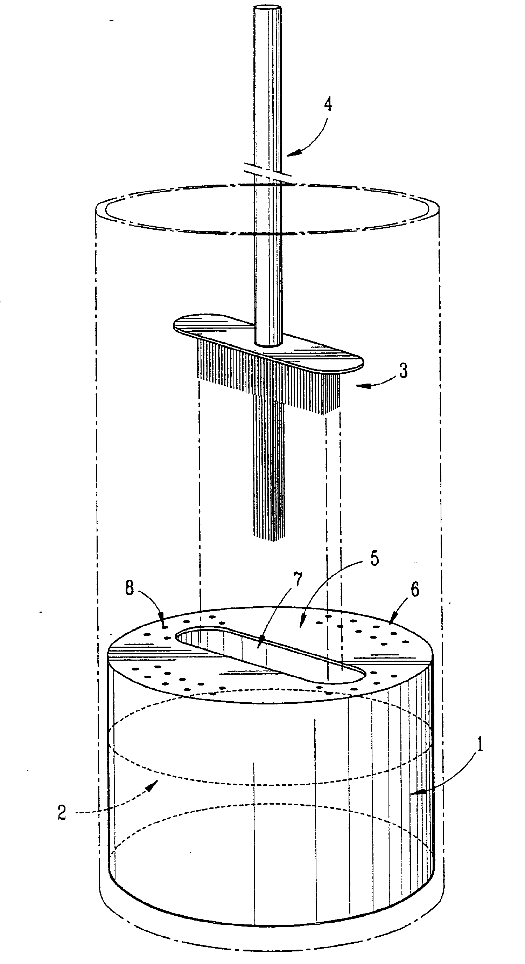

L'invention sera mieux comprise à la lecture de la description qui va suivre, donnée uniquement à titre d'exemple et faite en se référant au dessin annexé qui représente une vue en perspective d'un exemple de réalisation d'un flacon selon l'invention.

On a en effet représenté sur cette figure, un exemple de réalisation d'un flacon 1 de préparation d'une suspension cytologique, par exemple cervicale ou vaginale.

Ce flacon est destiné à recevoir un fixateur cellulaire désigné par la référence générale 2 et une brosse de prélèvement désignée par la référence générale 3, fixée de façon détachable sur un manche de manipulation 4, de façon classique.

Cette brosse, après prélèvement, est introduite dans le flacon à travers une extrémité ouverte 5 de celui-ci.

Selon l'invention, cette extrémité ouverte 5 du flacon comporte des moyens de butée pour la brosse, permettant de bloquer celle-ci dans le flacon une fois que le praticien l'y a introduite, et donc de la détacher du manche, par exemple par une simple traction sur celui-ci, afin que la brosse tombe dans le fixateur.

Ceci évite alors toute manipulation de la brosse par le praticien au moment où il la dépose dans le flacon et réduit ainsi les risques de contamination de la suspension par des squames, etc..

De plus, cette extrémité du flacon comporte également au moins une portion de voile ajourée permettant de filtrer la suspension lors d'un versement, en retenant les débris les plus importants.

Dans l'exemple de réalisation représenté sur cette figure, l'extrémité ouverte 5 du flacon comporte en fait un voile transversal 6 qui est muni d'un trou 7, par exemple centré sur l'axe du flacon et qui permet le passage de la brosse de prélèvement 3 en vue de son introduction dans le flacon.

En fait, la brosse 3 et le trou correspondant 7 du voile 6, peuvent présenter des formes complémentaires allongées, ce qui permet l'introduction de la brosse dans le flacon selon une orientation angulaire déterminée.

Si le praticien fait tourner légèrement la brosse dans le flacon, celle-ci peut alors venir en butée contre les bords du voile 6 délimitant ce trou 7, afin de bloquer la brosse 3 dans le flacon et de la détacher du manche 4 en tirant sur celui-ci.

Le praticien n'est donc plus amené à toucher la brosse ou le manche à proximité de celle-ci lorsqu'il souhaite la détacher de ce manche.

Par ailleurs, une telle structure est dite à usage unique en rendant plus difficile la récupération de la brosse en vue d'une nouvelle utilisation de celle-ci comme cela pouvait se pratiquer avec les risques que cela comportait.

Dans l'exemple décrit sur cette figure, des ajourages 8 sont également prévus dans le voile 6 autour du trou 7 de celui-ci.

Ces ajourages sont par exemple régulièrement répartis autour de ce trou.

Ceci permet comme cela a été indiqué précédemment, de filtrer la suspension lors d'un versement.

Ce flacon peut par exemple être réalisé en matière plastique ou autre.

## Revendications

1. Flacon de préparation d'une suspension cytologique à base de fixateur, ce flacon (1) étant muni d'une ouverture (5) destinée à recevoir une brosse de prélèvement cytologique (3) fixée de façon détachable sur un manche de manipulation (4), l'ouverture (5) du flacon comportant des moyens de butée (6) pour la brosse, permettant de bloquer celle-ci dans le flacon et de la détacher du manche (4), et d'un voile (6) obturant l'ouverture (5) du flacon, ledit voile étant muni d'un trou (7) de passage de la brosse de prélèvement (3), les bords qui délimitent ce trou formant lesdits moyens de butée, **caractérisé en ce qu'**au moins une portion du voile (6) est ajourée (8) pour filtrer la suspension lors du versement.

2. Flacon selon la revendication 1, **caractérisé en ce que** le trou (7) du voile est centré sur l'axe du flacon (1).

3. Flacon selon l'une quelconque des revendications précédentes, **caractérisé en ce que** des ajourages de filtrage (8) sont régulièrement répartis dans le voile (6) de l'ouverture du flacon autour du trou (7) de celui-ci.

4. Flacon selon l'une quelconque des revendications précédentes, **caractérisé en ce que** la brosse de prélèvement (3) et le trou (7) du voile présentent des formes allongées complémentaires.

5. Flacon selon l'une quelconque des revendications précédentes, **caractérisé en ce que** la suspension est une suspension cytologique cervicale.

6. Flacon selon l'une quelconque des revendications 1 à 4, **caractérisé en ce que** la suspension est une suspension cytologique vaginale.

## Claims

1. Flask for preparing a fixative-based cytological suspension, this flask (1) being provided with an opening (5) which is intended for receiving a brush (3) for cytological sampling, which brush is detachably fastened to a manipulation handle (4), the opening (5) of the flask comprising abutment means (6) for the brush, which means enable the brush to be locked in the flask and to be detached from the handle (4), and with a screen (6) which seals off the opening (5) of the flask, said screen being provided with a hole (7) for the passage of the sampling brush (3), the lips which delimit this hole forming said abutment means, **characterized in that** at least one portion of the screen (6) is perforated (8) for filtering the suspension during pouring.

2. Flask according to Claim 1, **characterized in that** the hole (7) of the screen is centred on the axis of the flask (1).

3. Flask according to any one of the preceding claims, **characterized in that** filtration perforations (8) are regularly distributed in the screen (6) of the opening of the flask around the hole (7) of this screen.

4. Flask according to any one of the preceding claims, **characterized in that** the sampling brush (3) and the hole (7) of the screen exhibit complementary oblong shapes.

5. Flask according to any one of the preceding claims, **characterized in that** the suspension is a cervical cytological suspension.

6. Flask according to any one of Claims 1 to 4, **characterized in that** the suspension is a vaginal cytological suspension.

## Patentansprüche

1. Fläschchen zur Herstellung einer zytologischen Suspension auf Grundlage eines Fixiermittels, wobei dieses Fläschchen (1) eine Öffnung (5) aufweist, welche zur Aufnahme einer zytologischen Entnahmebürste (3) vorgesehen ist, die an einem Handhabungshalter (4) entfernbar befestigt ist, wobei die Öffnung (5) des Fläschchens Anschlagmittel (6) für die Bürste, welche eine Arretierung dieser in dem Fläschchen und ihre Entfernung von dem Halter (4) ermöglichen, und eine Abdeckung (6) aufweist, welche die Öffnung (5) des Fläschchens verschließt, wobei die Abdeckung mit einem Durchgangsloch (7) für die Entnahmebürste (3) versehen ist, wobei die Ränder, welche diese Ausnehmung begrenzen, die Anschlagmittel bilden, **dadurch gekennzeichnet, dass** zumindest ein Abschnitt der Abdeckung (6) zur Filterung der Suspension beim Ausgießen Durchbrechungen (8) aufweist.

2. Fläschchen nach Anspruch 1, **dadurch gekennzeichnet, dass** das Loch (7) der Abdeckung auf der Mittellinie des Fläschchens (1) zentriert ist.

3. Fläschchen nach einem der vorhergehenden Ansprüche, **dadurch gekennzeichnet, dass** die Filterdurchbrechungen (8) auf der Abdeckung (6) der Öffnung des Fläschchens um dessen Loch (7) herum regelmäßig verteilt sind.

4. Fläschchen nach einem der vorhergehenden Ansprüche, **dadurch gekennzeichnet, dass** die Entnahmebürste (3) und das Loch (7) der Abdeckung komplementäre langgestreckte Formen aufweisen.

5. Fläschchen nach einem der vorhergehenden Ansprüche, **dadurch gekennzeichnet, dass** die Suspension eine zytologische Zervikalsuspension ist.

6. Fläschchen nach einem der Ansprüche 1 bis 4, **dadurch gekennzeichnet, dass** die Suspension eine zytologische Vaginalsuspension ist.
